# EUROPÄISCHE PATENTSCHRIFT

(11) **EP 2 440 267 B1**
(45) Veröffentlichungstag und Bekanntmachung des Hinweises auf die Patenterteilung: **24.10.2018**
(21) Anmeldenummer: 10722370.3
(22) Anmeldetag: 07.06.2010
(51) Int. Cl.: A61M 5/158

(54) **PORTKANÜLE MIT NADELSTICHSCHUTZVORRICHTUNG**
PORT NEEDLE HAVING NEEDLE-PRICK PROTECTION DEVICE
CANULE POUR CHAMBRE IMPLANTABLE, À DISPOSITIF DE PROTECTION CONTRE LA PIQÛRE PAR L'AIGUILLE

(30) Priorität: 10.06.2009 DE 102009025056; 16.02.2010 EP 10153708
(43) Veröffentlichungstag der Anmeldung: 18.04.2012
(73) Patentinhaber: Fresenius Kabi Deutschland GmbH, 61352 Bad Homburg (DE)
(72) Erfinder: HORNIG, Wolfgang, 58566 Kierspe-Bollwerk (DE); OSTER, Marcel, 41065 Mönchengladbach (DE); MAURER, Jürgen, 61381 Friedrichsdorf (DE); PAPIOREK, Martina, 65597 Hünfelden (DE); PECH, Bernhard, 65510 Hünstetten (DE)
(74) Vertreter: Kusche, Robert
(86) Internationale Anmeldenummer: PCT/EP2010/057926
(87) Internationale Veröffentlichungsnummer: WO 2010/142641

(56) Entgegenhaltungen:
- WO-A2-02/055132
- WO-A2-2008/092958
- US-A1- 2003 168 366
- US-A1- 2004 199 112

## Beschreibung

### Technisches Gebiet

Die vorliegende Erfindung betrifft eine Portkanüle aufweisend eine Auflage zur Auflage der Portkanüle am Patienten, eine Zuführung und eine Kanüle mit einer Spitze.

Solche Portkanülen sind im Stand der Technik zahlreich vorhanden und werden genutzt, um eine Verbindung zu einem im menschlichen oder tierischen Körper implantierten Port herzustellen. Solche Ports werden implantiert, um einfacher und zuverlässiger Substanzen in den menschlichen Körper, insbesondere in zentrale Gefäße, einzubringen.

Zur Verbindung zwischen Portkanüle und Port weist eine Portkanüle eine Kanüle mit Kanülenspitze auf. Eine solche Kanülenspitze ist gefährlich und daher im Auslieferungszustand in der Regel durch einen Kanülenschutz umschlossen. Dieser wird kurz vor Applizierung der Portkanüle entfernt. Die Kanüle ist zu diesem Zeitpunkt steril und stellt zwar ein Verletzungs-, aber kein Infektionsrisiko für den Anwender dar.

Wird die Portkanüle hingegen aus dem Patienten bzw. aus dem Port des Patienten entfernt, ist sie nicht mehr steril und stellt somit nicht nur ein Verletzungs-, sondern auch ein hohes Infektionsrisiko nicht nur für das medizinische Personal, sondern auch für das Personal der Abfallwirtschaft dar. Aus diesem Grund ist es wünschenswert, Portkanülen mit einer Nadelstichschutzvorrichtung auszurüsten, die insbesondere beim und nach Entfernen der Portkanüle nach der Anwendung vor Stichverletzungen schützt. Dies ist insbesondere auch aufgrund steigender Sicherheitsanforderungen notwendig.

Bei der Entwicklung eines Nadelstichschutzes stehen verschiedene Aspekte in Konkurrenz zueinander: einerseits ist es wichtig, dass der Greifbereich für das medizinische Personal beim Anlegen einer Kanüle groß und sicher zu fassen ist und der Sicherheitsmechanismus so robust ist, dass er bei der Anlage nicht ausgelöst wird. Ein hoher Aufbau des Griffes mit aufwändiger Sicherheitstechnik erniedrigt jedoch den Tragekomfort der Kanüle für den Patienten.

### Stand der Technik

So offenbart beispielsweise die US 6,238,375 einen niedrigen Aufbau eines Nadelstichschutzes, bei dem die Nadel an einem schmalen Griffteil herausgezogen wird, gleichzeitig zwei Schutzelemente teleskopartig auseinanderfahren und somit die Nadel verbergen. Bei diesem Ansatz ist der schmale Nadelhalter bzw das Griffteil an dem oberen Teleskopsegment angebracht. Die Teleskopsegmente werden nur in Zugrichtung am Durchgleiten gehindert, nicht jedoch in die Ausgangsrichtung. Der Nadelschutz wird durch eine herunterfallende Bodenplatte gewährleistet, wobei die Bodenplatte jedoch konstruktionsbedingt in der Mitte geschlitzt ist und somit einem Druck von oben nicht standhalten könnte.

Die WO03028784 offenbart eine stabile, gut greifbare Konstruktion einer geschützten Portkanüle, die jedoch einen hohen Aufbau mit Ecken und Kanten besitzt und daher für den Patienten unbequem zu tragen ist, da er leicht an den hohen Aufbau stößt oder daran hängen bleibt, was einerseits schmerzhaft sein kann und andererseits den Sitz der Portkanüle beeinträchtigen kann.

Eine Portkanüle Vorrichtung gemäß dem Oberbegriff des Anspruch 1 ist auch aus dem Dokument WO2008/092958 A2 bekannt.

### Darstellung der Erfindung

Eine erfindungsgemäße Portkanüle weist neben einer Auflage zur Auflage der Portkanüle am Patienten, also am Patienten selbst bzw. am beim Menschen oder Tier implantierten Port, einer Zuführung, die beispielsweise aus einem Schlauch oder einem Anschluss für einen solchen bestehen kann, und einer Kanüle mit einer Spitze einen Sicherungsabschnitt auf. Dabei kann die Kanüle mit ihrer Spitze mindestens in zwei Positionen, eine Punktionsstellung und eine zurückgezogene Stellung verbracht werden. In der Punktionsstellung ragt die Nadel mit ihrer Spitze weit heraus. Eine Portkanüle wird in der Regel in einer solchen Punktionsstellung ausgeliefert, wobei dann die Kanüle durch einen Kanülenschutz umgeben ist. Zur Anwendung wird der Kanülenschutz entfernt. Dann kann die Portkanüle, die sich in der Punktionsstellung befindet, direkt in den Port eingestochen werden. In der zurückgezogenen Stellung wird die Kanülenspitze erfindungsgemäß durch den Sicherungsabschnitt abgeschirmt.

Dies bietet den schon eingangs angesprochenen Vorteil, dass das Personal nun vor Nadelstichen und Infektionen geschützt ist. Dabei muss der Sicherungsabschnitt die Kanüle nicht vollständig umschließen, sondern nur so eingerichtet sein, dass bei vernünftiger Handhabe die Kanülenspitze ausreichend abgeschirmt ist. Dies ist beispielsweise auch dann gegeben, wenn der Sicherungsabschnitt nach unten offen ist, die Kanülenspitze jedoch soweit in ihn verbracht wird, dass beim Kontakt mit der menschlichen Haut eine Berührung und insbesondere Verletzung der Haut mit der Spitze der Kanüle auszuschließen ist. Dies hängt nicht nur von der Tiefe der Verbringung der Kanülenspitze, sondern auch von der Öffnungsweite des Sicherungsabschnittes ab. Geeignete Dimensionen lassen sich vom Fachmann ohne weiteres leicht auffinden.

Die Kanüle ist vorzugsweise an einem Kanülenhalter befestigt. Bevorzugt wird der Kanülenhalter durch mindestens eine Verbindung, vorzugsweise ein Rastelement, besonders bevorzugt mindestens zwei Rastelemente oder Gleit- oder Halteelemente, mit einem mit der Auflage verbundenen Element oder Vorrichtung zur Arretierung des Kanülenhalters lösbar verbunden. Die Lösung der Verbindung oder Verrastung erfolgt durch einen Zug entlang der Kanülenachse. Die notwendige Kraft zur Lösung der Verbindung oder Verrastung kann beispielsweise im Falle einer Verrastung über den Rastwinkel der Rastelemente, die Breite der Rastelemente, die Gestaltung der Geometrie-Paarung sowie die Flexibilität und die Materialkombination beeinflusst und festgelegt.

Für die kontrollierte Anlage der Portnadel ist es von großer Bedeutung, dass der Sicherheitsmechanismus nicht vorzeitig ausgelöst wird, damit auch im Fall einer Lagekorrektur, die im Extremfall sogar eine Entfernung aus der Portmembran und Wiederanlage bedeuten kann, die Portkanüle sicher und kontrolliert gehandhabt werden kann. Hierzu kann beispielsweise eine Verrastung vorgesehen sein, die durch Lösen eines Mechanismus, beispielsweise eines Hebels, gelöst werden kann. Bevorzugt ist es jedoch, wenn der Sicherheitsmechanismus durch einen Zug entlang der Kanülenachse ausgelöst wird, wobei die Kraft, die nötig ist, um den Sicherheitsmechanismus auszulösen, 5 N oder mehr beträgt. Besonders bevorzugt ist es, wenn eine Kraft von mindestens 8 N nötig ist, vorzugsweise ≥8 bis ≤13 N, ganz besonders bevorzugt von ≥9 bis ≤11 N.

Die lösbare Verbindung des Kanülenhalters mit dem mit der Auflage verbundenen Element oder der Vorrichtung zur Arretierung des Kanülenhalters, die durch eine Kraft von 5 N oder mehr, bevorzugt von 8 N oder mehr, besonders bevorzugt von ≥ 8 bis ≤13 N, ganz besonders bevorzugt von ≥ 9 bis ≤ 11 N, kann auch durch andere Mittel als durch Rast- Gleit- oder Halteelemente erreicht werden, beispielsweise durch abzubrechende Stege, Klebemittel oder Steckverbindungen.

Um mit der nötigen Kraft zur Auslösung des Sicherheitsmechanismus auf die Portkanüle einwirken zu können ist es vorteilhaft, wenn die Auflage der Portkanüle breiter ist als die mit der Auflage verbundene Vorrichtung zur Arretierung des Kanülenhalters. Damit kann die Auflage durch Druck fixiert werden, was die Auslösung des Sicherheitsmechanismus erleichtert und verhindert, dass die Portkanüle ohne Auslösen des Sicherheitsmechanismus aus dem Port entfernt wird.

Vorteilhafterweise weist die Auflage ein Höhenausgleichselement auf, das zwischen Auflage und Kanüle positioniert, dehnbar und/oder flexibel ist.

Ein solches Höhenausgleichselement ist nicht nur geeignet, kleine Relativbewegungen zwischen Auflage und Kanüle zu erlauben, die entstehen, wenn sich der Patient bewegt, sondern auch in der Lage, Höhenunterschiede und Schwankungen zwischen Portboden und Auflage auszugleichen. Zwar existieren Portkanülen mit unterschiedlich langen Kanülen, durch die eine Anpassung der Kanülenlänge auf die individuelle Einbausituation des Ports beim Patienten möglich ist, jedoch sind solche Portkanülen nur in einer diskreten Längenstaffelung von wenigen Millimetern praktikabel vorzuhalten. Darüber hinaus, kann die Einbautiefe des Ports im Zeitablauf oder bei Bewegung des Patienten kleinen Schwankungen unterworfen sein. Optimalerweise reicht die Kanüle aber genau bis auf den Portboden. Somit ist es durch ein Höhenausgleichselement möglich, eine optimale Lage der Kanülenspitze zu gewährleisten und den Tragekomfort zu erhöhen.

Dabei kommt es nicht darauf an, dass das Höhenausgleichselement direkt mit der Auflage und/oder der Kanüle verbunden ist. Die Verbindung kann vielmehr durch weitere Elemente vermittelt sein. Es kommt lediglich darauf an, dass keine steife Verbindung zwischen Auflage und Kanüle besteht. Das Höhenausgleichelement kann dabei beispielsweise aus einem flexiblen Ring, bei dem der Höhenausgleich durch eine wellenförmige Ausführung gewährleistet sein kann, oder einer dehnbaren Zwischenlage bestehen. Prinzipiell wären zwar auch kompressible Elemente geeignet, eine Relativbewegung zwischen Auflage und Nadel zu ermöglichen, doch eignen sich solche nur sehr bedingt, das sie auch ein tieferes Eindringen der Kanüle in den Patienten zulassen und so zu Beschädigungen des Ports oder zu Verletzungen des Patienten führen können. Das Höhenausgleichselement kann beispielsweise geklebt, geklemmt oder angespritzt oder auch in Zweikomponententechnik gespritzt sein.

Vorzugsweise ist die Kanüle an einem Kanülenhalter befestigt, in dem vorzugsweise auch die Zuführung angebracht ist. Ein solcher Kanülenhalter schützt die Kanüle bei der Handhabung vor Bruch und ermöglicht dem Personal eine griffigere und sicherere Handhabe. Ein solcher Kanülenhalter ist vorteilhafterweise endständig zur Kanüle angebracht, da dann die Funktion als Handhabe besonders ausgeprägt ist. Wird auch die Zuführung in dem Kanülenhalter integriert, so kann innerhalb des Kanülenhalters eine zuverlässige und sichere Verbindung zwischen Zuführung und Kanüle realisiert werden.

Besonders vorteilhaft ist es, wenn erste Rastelemente vorgesehen sind, die die Kanüle in der zurückgezogenen Stellung verrasten. Solche ersten Rastelemente müssen nicht notwendiger Weise an der Kanüle selbst angebracht werden, sondern können auch an weiteren Elementen, wie beispielsweise dem Kanülenhalter, befestigt werden. Vorzugsweise ist die Verrastung irreversibel oder kann nur durch Ausübung von einem Druck von mindestens 30 N gelöst werden. Durch eine solche Verrastung kann sichergestellt werden, dass ein versehentliches Verändern der Kanülenposition nach dem Verbringen in die zurückgezogene Stellung und somit ein Verbringen der Kanüle aus dem Sicherungsabschnitt heraus nicht möglich ist.

Vorteilhafterweise sind zweite Rastelemente vorgesehen, die eine Verrastung in der Punktionsstellung ermöglichen. Durch eine solche Verrastung in der Punktionsstellung wird das Punktieren erleichtert, insbesondere dann, wenn die Kraft, die zur Lösung der Verrastung nötig ist, mindestens 5 N beträgt, vorzugsweise mindestens 8 N, besonders bevorzugt ≥ 8 bis ≤13 N, ganz besonders bevorzugt ≥ 9 bis ≤11 N.

Der Kanülenhalter, der zugleich als Griff dienen kann, weist vorteilhafterweise einen umlaufenden Führungs- und Halterand auf, der ein sicheres Positionieren, Repositionieren und Lösen der Kanüle ermöglicht.

Bevorzugt weist die erfindungsgemäße Portkanüle eine Basis auf, die mit der Auflage verbunden ist und relativ zu der die Kanüle in die mindestens zwei Positionen, die Punktionsstellung und die zurückgezogene Stellung, verbringbar ist. Eine solche Basis bietet den Vorteil, dass eine griffige Handhabe und/oder eine stabile Verankerungsmöglichkeit für die weiteren Elemente geschaffen wird/werden. Es ist dabei durchaus vorstellbar, dass die Basis und die Auflage sowie weitere Teile einteilig hergestellt sind. Auch kann das Höhenausgleichselement in der Basis integriert werden oder diese bilden.

Vorteilhafterweise weist eine erfindungsgemäße Portkanüle eine Klebeplatte zur Befestigung der Portkanüle bzw. der Auflage am Patienten auf. Dies hat den Vorteil, dass die Portkanüle nach der Anlage nicht überklebt werden muss, sondern mit der Klebeplatte direkt auf der Haut befestigt werden kann. Eine solche Klebeplatte kann auf verschiedenste Weise ausgeführt werden. Sie kann beispielsweise zwischen Auflage und Patient angebracht werden oder um die Auflage herum angeordnet sein. Sie kann bereits mit dem Höhenausgleichselement verbunden vorgefertigt werden oder passgenau mit der Portkanüle mitgeliefert und nach der Anlage aufgeklebt werden. Die Klebeplatte wird bevorzugt aus einem hautfreundlichen Material mit antiallergenem Klebematerial bereitgestellt. In einer bevorzugten Ausführungsform ist das Gewebematerial durchsichtig. Weiterhin kann die Klebeplatte eine zusätzliche Lasche aufweisen, die nach der Anlage der Portkanüle zur zusätzlichen Sicherung über die Kanüle geklebt werden kann.

Vorteilhafterweise wird die erfindungsgemäße Portkanüle derart ausgeführt, dass die Portkanüle in der Punktionsstellung eine geringere Höhe aufweist als in der zurückgezogenen Stellung. Besonders vorteilhaft ist es, wenn die Höhe in der Punktionsstellung geringer ist als die Erstreckung der Kanüle in Punktionsrichtung. Dabei ist unter Punktionsrichtung die Richtung zu verstehen, in der die Punktion erfolgt, in der Regel also senkrecht zur Auflagefläche. Dies ist besonders vorteilhaft, da die Portkanüle auf der Haut des Patienten unter Umständen auch für längere Zeit verbleibt. Dann befindet sich die Portkanüle in der Punktionsstellung. Daher ist es günstig, wenn die Portkanüle in der Punktionsstellung eine besonders geringe Höhe aufweist. Unter Höhe ist dabei die Erstreckung der Portkanüle zu verstehen, die zwischen Auflage und dem von der Auflage in Richtung vom Patienten weg weitest entfernten Punkt liegt. Eine geringe Höhe erhöht somit den Tragekomfort und verringert das Risiko des Verwackelns oder Herausreißens der Portkanüle.

Andererseits muss der Kanülenhalter noch sicher vom Anwender gegriffen werden können. Vorzugsweise weist die Portkanüle in der Punktionsstellung eine Höhe von ≥ 0,8 cm und ≤ 1,8 cm, besonders bevorzugt von ≥ 1 cm und ≤ 1,5 cm auf.

Eine vorteilhafte Ausführungsform weist mindestens zwei Teleskopsegmente auf, die gegeneinander beweglich und untereinander geführt ausgebildet sind. Dabei ist die Kanüle mit einem der Teleskopsegmente verbunden und ein anderes Teleskopsegment ist mit der Auflage verbunden. Eine Verbindung der Kanüle mit einem Teleskopsegment muss nicht unbedingt direkt erfolgen, sondern kann beispielsweise über den Kanülenhalter vermittelt werden. Ähnliches gilt für die Verbindung mit der Auflage, die beispielsweise über die Basis hergestellt werden kann. Bei einer solchen Ausführung der Teleskopsegmente sind die Teleskopsegmente in der Punktionsstellung weitestgehend ineinander geschoben und in der zurückgezogenen Stellung weiter auseinander gezogen. Eine solche Ausführung als Teleskop ermöglicht eine sehr robuste Ausführung bei geringer Bauhöhe, die auch in der zurückgezogenen Stellung eine hohe Steifigkeit aufweist. Des Weiteren ist es möglich, die Kanüle in der zurückgezogenen Stellung vollständig mit Teleskopsegmenten zu umschließen, so dass ein Bruch der Kanüle, aus dem weitere Verletzungsgefahren entstehen könnten, zuverlässig verhindert werden kann. Vorteilhafterweise sind die Teleskopsegmente so ineinander geschachtelt, dass das innerste Teleskopsegment mit der Kanüle bzw. dem Kanülenhalter verbunden ist. Eine solche Verbindung kann beispielsweise durch Verrastung oder Verklebung erfolgen. Kanülenhalter und innerstes Teleskopsegment können aber auch durch das gleiche Element gebildet werden. Besonders vorteilhaft ist es dabei, wenn der Kanülenhalter wiederum so ausgebildet ist, dass er in der Punktionsstellung alle Teleskopsegmente zumindest teilweise umschließt. Dadurch kann in der Punktionsstellung eine für die Punktion förderliche besonders hohe Stabilität erzielt werden.

Vorteilhafterweise ist ein Teleskopsegment mit der Basis verbunden bzw. bildet ein Teleskopsegment die Basis und/oder ist eines der Teleskopsegmente mit dem Kanülenhalter verbunden bzw. bildet eines der Teleskopsegmente den Kanülenhalter. Eine solche Ausführung ermöglicht eine möglichst kompakte und robuste Ausführung. Bei der Verwendung eines Höhenausgleichselements ist dabei darauf zu achten, dass das Höhenausgleichselement vorteilhafterweise zwischen Auflage und dem zur Auflage hin angeordneten Teleskopelement angeordnet wird, um die Lage der Kanüle relativ zu den Teleskopsegmenten definiert zu halten und eine optimale Wirkung des Höhenausgleichselements in der Punktionsstellung zu gewährleisten.

Bevorzugt wird der Kanülenhalter durch mindestens ein lösbares Rastelement, vorzugsweise mindestens zwei lösbare Rastelemente oder auch Gleit- oder Halteelemente am Kanülenhalter sowie dem feststehenden unteren Teleskopelement verrastet. Die Lösung der Verrastung erfolgt durch einen Zug entlang der Kanülenachse. Die notwendige Kraft zur Lösung der Verrastung wird über den Rastwinkel der Rastelemente, die Breite der Rastelemente, die Gestaltung der Geometrie-Paarung sowie die Flexibilität und die Materialkombination beeinflusst und festgelegt.

Für die kontrollierte Anlage der Portnadel ist es von großer Bedeutung, dass der Sicherheitsmechanismus nicht vorzeitig ausgelöst wird, damit auch im Fall einer Lagekorrektur, die im Extremfall sogar eine Entfernung aus der Portmembran und Wiederanlage bedeuten kann, die Portkanüle sicher und kontrolliert gehandhabt werden kann. Hierzu ist es bevorzugt, wenn die Kraft, die nötig ist, um den Sicherheitsmechanismus auszulösen, 5N oder mehr beträgt, vorzugsweise mindestens 8 N. Besonders bevorzugt ist es, wenn eine Kraft von ≥ 8 bis ≤ 13 N nötig ist, ganz besonders bevorzugt von ≥ 9 bis ≤ 11 N.

Es ist vorteilhaft, wenn der Kanülenhalter geschlossene, verrundete Flächen zum Greifen aufweist.

Vorteilhafterweise weist die Portkanüle an jedem Teleskopsegment mindestens ein erstes Rastelement für die Verrastung in der zurückgezogenen Stellung auf. Diese ersten Rastelemente können auch gemeinsam mit oberen Begrenzungen gegen zu weites Ausziehen ausgeführt sein. Die ersten Rastelemente, die das Zurückschieben verhindern, können beispielsweise durch Rastlaschen und/oder Rastnasen, die mit der Unterkante oder Oberkante des nächsten Elements zusammenwirken, gebildet werden. Es ist von Vorteil, wenn die Rastelemente so ausgebildet sind, dass der Sicherheitsmechanismus einem Druck von mindestens 30 N standhält, vorzugsweise einem Druck von mindestens 40 N standhält, damit der Sicherheitsmechanismus nicht durch Unachtsamkeit gelöst werden kann.

In einer bevorzugten Ausführungsform sind die einzelne Teleskopelemente mit einer im wesentlichen umlaufenden, innen liegenden Stopprippe versehen, sodass das Durchziehen der Segmente bei einem Zug von bis zu 30 N, vorzugsweise bis zu 35 N nicht möglich ist.

Die Vorteile einer solchen Verrastung wurden schon ausführlich beschrieben und zielen insbesondere darauf ab, ein versehentliches Verbringen der Kanüle aus der zurückgezogenen Stellung heraus zu verhindern.

Vorteilhafterweise weist eine erfindungsgemäße Portkanüle an jedem Teleskopsegment mindestens ein erstes Rastelement für die Verrastung mit jedem benachbartem Teleskopsegment auf, um so zuverlässig die Verrastung in der zurückgezogenen Stellung auf einfache Weise zu sichern. Mittlere Teleskopsegmente weisen somit erste Rastelemente zur Verrastung mit zwei weiteren Teleskopsegmenten auf. Dabei können erste Rastelemente teilweise auch durch ohnehin vorhandene Kanten und Flächen gebildet werden beziehungsweise mit diesen zusammenwirken. Das erste und das letzte bzw. innerste und äußerste Teleskopsegment weisen somit nur erste Rastelemente für die Verrastung mit einem weiteren Teleskopsegment auf.

Bevorzugt weisen die ineinander geschachtelten Teleskopsegmente ein so geringes Spiel auf und sind die sich aneinander vorbei bewegenden Wände der Teleskopsegmente so geformt und so präzise ausgeführt, dass ein Verdrehen des Kanülenhalters und/oder Verkanten der Nadel beim Bewegen in die zurückgezogenen Stellung und/oder in der zurückgezogenen Stellung verhindert wird.

Bei einer solchen Auslegung ist zwar darauf zu achten, dass sich die geschachtelten Teleskopsegmente ohne großen Widerstand teleskopartig gegeneinander bewegen lassen, doch kann das Spiel und insbesondere die Ausrichtung der Wandungen so gewählt werden, dass eine ausschließlich lineare Bewegung der Teleskopsegmente sichergestellt ist. Vorzugsweise sind die Teleskopsegmente als Hohlquader ausgebildet, bei denen zwei gegenüberliegende und sich senkrecht zur Längserstreckung der Kanüle erstreckende Seitenflächen ausgespart sind. In dieser Ausführung lassen sich die Teleskopelemente besonders präzise in linearer Richtung bewegen. Dabei sollte sich diese lineare Bewegungsrichtung senkrecht zur Auflagefläche erstrecken. Dies unterstützt den Anwender beim exakten Zurückziehen der Kanüle.

Bei einer Ausführung der Teleskopelemente als Rundrohrstücke sind vorteilhafterweise weitere Vorkehrungen gegen ein Verdrehen vorgesehen.

Vorteilhafterweise ist die Kanüle innerhalb der Teleskopsegmente angeordnet. Dies bedeutet, dass die Kanüle in der zurückgezogenen Stellung seitlich vollständig von Teleskopsegmenten umgeben ist. Wird die Kanüle jedoch aus der zurückgezogenen Stellung heraus bewegt, ist sie nicht mehr vollständig innerhalb von Teleskopsegmenten angeordnet, sondern nur insofern innerhalb der Teleskopsegmente, wie sie sich noch nicht in axialer Richtung aus diesen herausbewegt hat.

Vorteilhafterweise sind vier Teleskopsegmente vorgesehen, bei denen das erste mit der Basis und das letzte mit dem Kanülenhalter verbunden ist. Die Verwendung von vier Teleskopsegmenten hat sich als besonders günstiger Kompromiss zwischen Stabilität und Höhe der Portkanüle in Punktionsstellung erwiesen. Dabei ist es von besonderem Vorteil, wenn als Teleskopsegemente Hohlquader zur Anwendung gelangen, bei denen zwei gegenüberliegende und sich senkrecht zur Längserstreckung der Kanüle erstreckende Seitenflächen ausgespart sind. Durch die Verwendung von solchen Hohlquadern kann bei geringer Baugröße eine besonders gute Stabilität gegen Verdrehung und Verkippen erzielt werden.

Alternativ können auch Verkippungsmittel vorgesehen sein, die die Kanüle beim Bewegen in Richtung der zurückgezogenen Stellung verkippen, so dass die Kanülenspitze nach einer gewissen, vorbestimmten Bewegung in Richtung der zurückgezogenen Stellung auf Grund des Zusammenspiels mit dem Sicherheitsabschnitt nicht wieder aus dem Sicherheitsabschnitt heraus in Richtung der Punktionsstellung bewegt werden kann. Dadurch kann sichergestellt werden, dass auch vor Eintritt der Verrastung in die zurückgezogene Stellung oder auch ohne Verrastungsmittel ein wirksamer Nadelstichschutz besteht. Das Verkippen kann dabei beispielsweise dazu führen, dass die Nadelspitze aus der Flucht mit einem Loch einer Blende herausgebracht wird und so nicht wieder durch dieses Loch hindurch in Richtung der Punktionsstellung bewegt werden kann. Dies kann beispielsweise durch schiefe Ebenen oder asymmetrische Ausbildung der einzelnen Teleskopsegmente erreicht werden. Verständlicherweise ist eine solche Verkippung erst dann wirkungsvoll, wenn sich die Kanülenspitze schon innerhalb des Sicherheitsabschnitts befindet. In der Regel ist dieser (zusätzliche) Schutzmechanismus somit nur relevant, wenn keine Verrastung vorgesehen ist oder die Kanülenspitze schon in den Sicherheitsabschnitt verbracht, die Verrastung aber noch nicht bewirkt wurde. Somit kann das Verkippen auch einen zusätzlichen Schutzmechanismus für den Fall des Versagens der Verrastung in zurückgezogener Stellung darstellen. In jedem Fall schützt es aber bei Fehlbedienungen, bei denen keine Verrastung bewirkt wird.

Mit Vorteil können die Verkippungsmittel durch unterschiedlich ausgebildete erste Rastelemente gebildet werden. Dies kann beispielsweise dadurch geschehen, dass bei einer mehrstufigen Verrastung mehrerer Teleskopsegmente die zuerst erfolgende Verrastung auch eine Verkippung der Nadel bewirkt, indem beispielsweise vorgesehene Rastlaschen eine unterschiedliche Länge aufweisen.

Vorteilhafterweise ist mindestens ein erstes Rastelement für die Verrastung in zurückgezogener Stellung und zur Verrastung von zwei Teleskopsegmenten, die beim Bewegen in die zurückgezogene Stellung vorzugsweise als letztes verrasten, so ausgebildet, dass es bei der Verrastung ein Geräusch abgibt, das sich von allen anderen Geräuschen der Verrastung der Portkanüle unterscheidet. Dies bedeutet, dass die Verrastung von zwei Teleskopsegmenten untereinander ein für die Portkanüle charakteristisches Geräusch erzeugt. Wird dieses charakteristische Geräusch bei der Verrastung der beim Bewegen in die zurückgezogene Stellung als letztes verrastenden Teleskopsegmente erzeugt, so kann dies für den Anwender das Zeichen sein, dass ein sicherer und nachhaltiger Nadelstichschutz erreicht wurde. Solch charakteristische Geräusche können beispielsweise durch eine massivere Auslegung von Rastlaschen erreicht werden. Dass die gewählten Teleskopsegmente als letztes verrasten, kann beispielsweise dadurch realisiert werden, dass zwischen ihnen eine höhere Reibung vorgesehen wird, als zwischen den anderen Teleskopsegmenten.

Vorteilhafterweise bildet das am nächsten zur Auflage hin, also am nächsten zum Patienten hin liegende Teleskopsegment den Sicherungsabschnitt. Dies bedeutet, dass die Kanülenspitze sich innerhalb dieses Teleskopsegmentes befindet, wenn sich die Kanüle in der zurückgezogenen Stellung steht. Darüber hinaus ist es erforderlich, dass die Kanülenspitze so weit in dieses Teleskopsegment hineingezogen wird, dass aufgrund des Zusammenspiels zwischen Öffnungsweite des Teleskopsegmentes bzw. einer ggf. vorhandenen Blende und der Entfernung der Kanülenspitze zu dieser Öffnung ein Berühren der Kanülenspitze zuverlässig verhindert werden kann.

Die Portkanüle liegt vor der Anwendung in einer Form vor, in der die Kanüle weit aus dem Nadelhalter ragt, die Teleskopsegmente ineinander geschachtelt und der Sicherheitsmechanismus verrastet ist. In einer bevorzugten Ausführungsform wird die Kanüle mit einer Schutzhülle versehen, die Nadelstichverletzungen bei der Vorbereitung der Anlage verhindern soll. Bevorzugt wird diese Schutzhülle nicht an der Bodenplatte eingeklemmt, sondern in eines der inneren Teleskopsegmente, um zu verhindern, dass beim Ziehen der Schutzhülle ein entgegengesetzter Zug auf Griffelement und Bodenplatte entsteht, der zum Auslösen des Sicherheitsmechanismus führen könnte.

Die erfindungsgemäße Portkanüle und das Sicherheitssystems können mit antimikrobiell wirkenden Additiven versehen und verarbeitet werden

Dieses Additiv ist in allen Kunststoffeinzelteilen einsetzbar, vorzugsweise jedoch in den Teilen, die das System umschließen. Diese sind: Griffstück (Kanülenhalter) sowie obere Halteplatte (feststehendes Segment, Bestandteil des Nivellierungssegmentes). Erreger, die sich von außen ansiedeln können, finden eine für sie lebensfeindliche Oberfläche vor und das Wachstum wird somit entscheidend gehemmt.

Die vorstehenden Ausführungen haben die Erfindung deutlich gemacht und ermöglichen es dem Fachmann, weitere Ausführungsformen aufzufinden, die der jeweiligen Anforderung angepasst sind. Die nun folgenden Ausführungsbeispiele dienen lediglich der Erläuterung weiterer Vorteile bzw. Umsetzungsmöglichkeiten, die auch dann einen zusätzlichen oder eigenständigen erfinderischen Beitrag leisten können, wenn sie nicht Gegenstand der nachfolgenden Ansprüche sind, und sollen den Schutzbereich keinesfalls einschränken. Die dazu verwendeten Zeichnungen sind rein schematisch und nicht beschränkend und zeigen im Einzelnen:

### Kurze Beschreibung der Abbildungen der Zeichnungen

Fig. 1 eine perspektivische Ansicht einer mit Teleskopsegmenten
Fig. 2 eine Detaildarstellung der Ansicht aus Fig. 1
Fig. 3 eine Schnittdarstellung einer Portkanüle in Punktionsstellung;
Fig. 4 eine im Vergleich zu Fig 3 gedrehte Schnittdarstellung einer Portkanüle in Punktionsstellung;
Fig. 5 Detaildarstellung der Elemente des Teleskopaufbaus;
Fig. 6,7,8 verschiedene Ausführungsformen der Gleitrippen 8b;
Fig. 9 Schnittdarstellung einer Portkanüle mit Teleskopelementen und ausgefahrenem Sicherheitsmechanismus
Fig. 10 Detailansicht einer Ausführungsform des Rastmechanismus 8;
Fig. 11 perspektivische Ansicht einer Portkanüle mit Teleskopelementen und ausgefahrenem Sicherheitsmechanismus
Fig. 12 Detailansicht einer Ausführungsform des Rastmechanismus 17;
Fig. 13 alternative Ausführung des Pflasters.
Fig.14 eine Schnittdarstellung einer Ausführungsform einer Portkanüle mit Stichschutz in Punktionsstellung, wobei der Sicherheitsmechanismus durch Lösen eines Hebels aktiviert wird.
Fig. 15 rückseitige Ansicht einer Portkanüle gemäß Fig. 14
Fig. 16 Detaildarstellung aus Fig 15
Fig 17 Portkanüle mit Hebel in zurückgezogener Stellung
Fig 18 Detaildarstellung aus Fig 17.

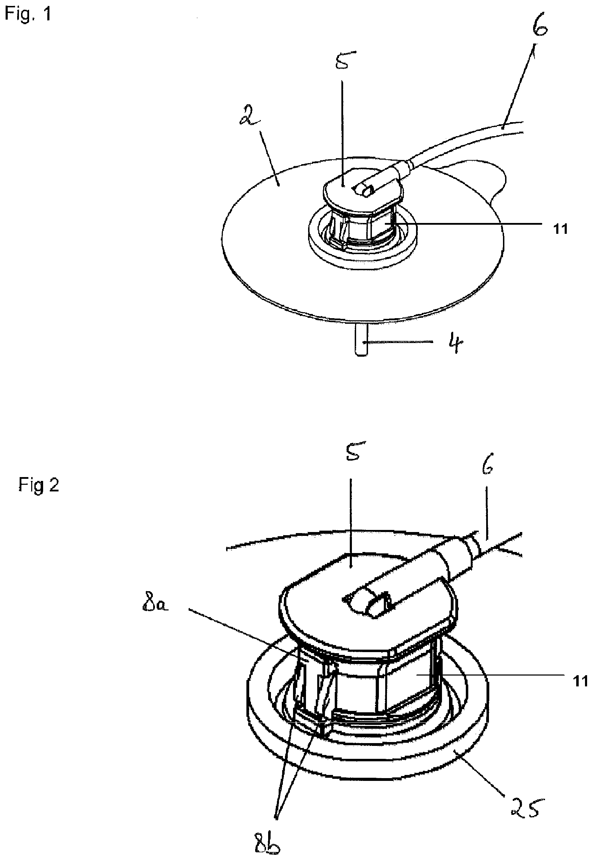

Die Figuren 1 bis 13 zeigen eine Ausführungsform mit Teleskopsegmenten 11 in Hohlquaderform. Eine solche Ausführung bietet bei geringer Baugröße eine hohe Stabilität sowohl in der Punktionsstellung als auch in der zurückgezogenen Stellung und in allen Zwischenpositionen. Darüber hinaus ist sie besonders verdrehsicher und bietet bei präziser Abstimmung der Teleskopsegmente 11 eine gute Führung der Kanüle.

Fig. 1 zeigt dabei eine Ansicht einer Portkanüle in Punktionsstellung mit dem im Auslieferungszustand vorhandenen Kanülenschutz 4. Des Weiteren zu erkennen sind der Schlauch 6, die Klebeplatte 2 und der Kanülenhalter 5, in der die Kanüle endständig befestigt ist, wobei eine Verbindung zwischen Kanüle und Schlauch 6 gegeben ist. Unter der Klebeplatte 2, die durch ein Pflaster gebildet wird, befindet sich eine mit der Basis verbundene Auflage.

Fig 2 zeigt die Verrastung des Sicherheitsmechanismus in der Punktionsstellung. In dieser Stellung wird der Kanülenhalter 5 mit dem ersten Teleskopelement 11 verrastet. Der durch Zug lösbare Mechanismus besteht aus einer beweglichen Klipslasche 8a und einem Gleitrippenpaar 8b, wobei sich der gezeigte Mechanismus auf zwei Seiten des Kanülenhalters 5 befindet.

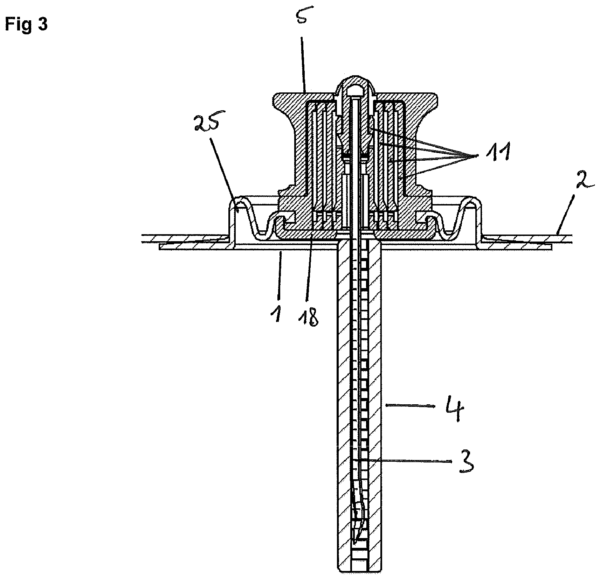

Fig. 3 zeigt eine Schnittdarstellung der Darstellung der Fig. 1. Zu erkennen sind neben der Kanüle 3 die Auflage 1 und die Klebeplatte 2. Die Auflage 1 ist mit dem Höhenausgleichselement 25 und der Basis 18 einteilig ausgeführt. Die Auflage 1 befindet sich unter der umlaufenden Klebeplatte 2. Des Weiteren zu erkennen sind Teleskopsegmente 11. In dem hier gezeigten Beispiel sind vier Teleskopsegmente 11 vorgesehen. Dabei ist das erste Teleskopsegment 11 mit dem Höhenausgleichselement 25 verbunden, und das vierte, innerste Teleskopsegment 11, das den geringsten Durchmesser aufweist, ist mit dem Kanülenhalter 5 verbunden. In der Punktionsstellung sind die Teleskopsegmente 11 alle ineinander eingefahren, so dass die Portkanüle eine geringe Höhe von etwa 1,5 cm zwischen Auflage 1 und oberstem Punkt des Kanülenhalters 5 aufweist. Dies ist besonders vorteilhaft, da bei der Punktion nur ein relativ kleines aber gut greifbares und in sich stabiles Teil vom Anwender gehandhabt werden muss. Darüber hinaus verbleibt die Portkanüle ggf. längere Zeit am Patienten, wobei ein wesentlicher Teil der Portkanüle auf der Hautoberfläche des Patienten angeordnet ist. Eine geringe Höhe ist somit förderlich für den Tragekomfort und verhindert darüber hinaus ein versehentliches Herausreißen oder Verwackeln der Portkanüle.

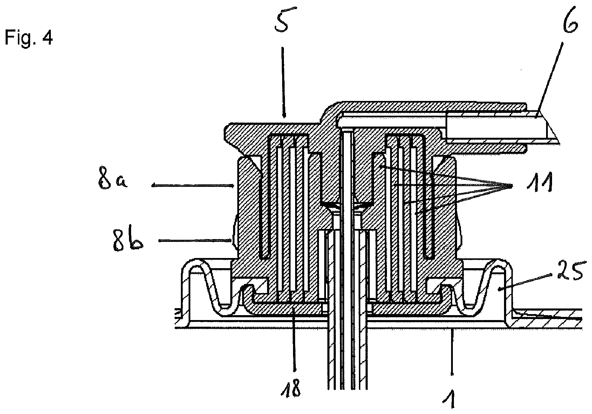

Fig. 4 zeigt ebenfalls eine Schnittdarstellung einer Portkanüle in einer Bauart gemäß der Darstellung der Fig. 1, jedoch mit gegenüber Fig. 3 um 90° gedrehter Schnittebene. Zu erkennen ist die Kanüle 3, die im Kanülenhalter 5 befestigt ist. Der Schlauch 6 ist mit dem Kanülenhalter verbunden. Zu erkennen sind hier wieder die vier Teleskopsegmente 11, von denen das äußerste und erste über das Höhenausgleichselement 25 mit der Basis 18 und der Auflage 1 verbunden ist. Das innerste, vierte und somit letzte Teleskopsegment 11 ist mit dem Kanülenhalter 5 verbunden ist. Der Kanülenhalter 5 weist die Gleitrippen 8b auf, die mit den Klipslaschen 8a am untersten Teleskopelement zusammenwirken. Durch diese Elemente 8a und 8b ist eine Verrastung in der Punktionsstellung möglich. Dabei sind zweite Rastelemente 17 zum einen an den Teleskopsegmenten 11 als auch am Kanülenhalter 5 angeordnet. Wird mit einer Kraft von ungefähr 9-12 N am Kanülenhalter gezogen bei gleichzeitiger Fixierung der Basis durch Druck auf die Klebeplatte oder die Höhenausgleichsplatte, so wird der Sicherheitsmechanismus ausgelöst.

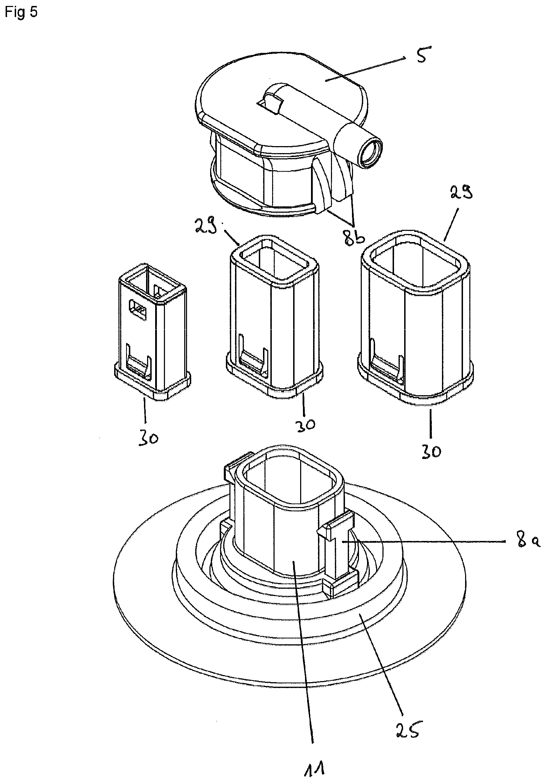

Fig 5 zeigt den Kanülenhalter 5, drei Teleskopelemente 11 und das untere Teleskopelement 11, das mit der Höhenausgleichsplatte verbunden ist. Die Gleitrippen 8b befinden sich am Nadelhalter, die Klipslaschen 8a an dem unteren Teleskopelement 11; darüber hinaus sind an den übrigen Teleskopelementen 11 die Rastelemente 17 zu erkennen.

Die Teleskopsegmente 11 weisen darüber hinaus obere Begrenzungen 29 auf. Diese oberen Begrenzungen 29 sind am oberen Ende der ersten drei Teleskopsegmente 11 angeordnet und wirken mit jeweils am unteren Ende der letzten drei der Teleskopsegmente 11 angebrachten unteren Anschlägen 30 zusammen. Obere Begrenzungen 29 und untere Anschläge 30 verhindern im Zusammenwirken ein zu weites Auseinanderziehen bzw Lösen der Teleskopsegmente.

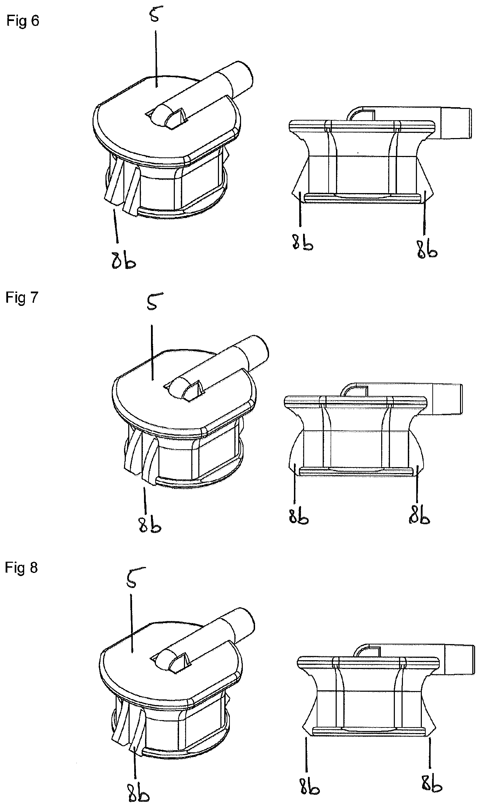

Fig 6, 7 und 8 zeigen unterschiedliche Geometrien der Gleitrippen. In Fig 6 sind die Rippen gerade. Das bedeutet, dass der Kraftverlauf für das Lösen des Sicherheitsmechanismus linear ist. In Fig 7 dagegen ist die Form der Gleitrippe konvex. Eine solche Form einer Gleitrippe erfordert eine hohe Anfangskraft beim Auslösen des Sicherheitsmechanismus. Fig 8 zeigt eine Ausführungsform mit konkavem Kraftverlauf, d.h., es wird eine geringe Anfangskraft benötigt, für das endgültige Entriegeln wird aber ein hohes Kraftaufkommen benötigt.

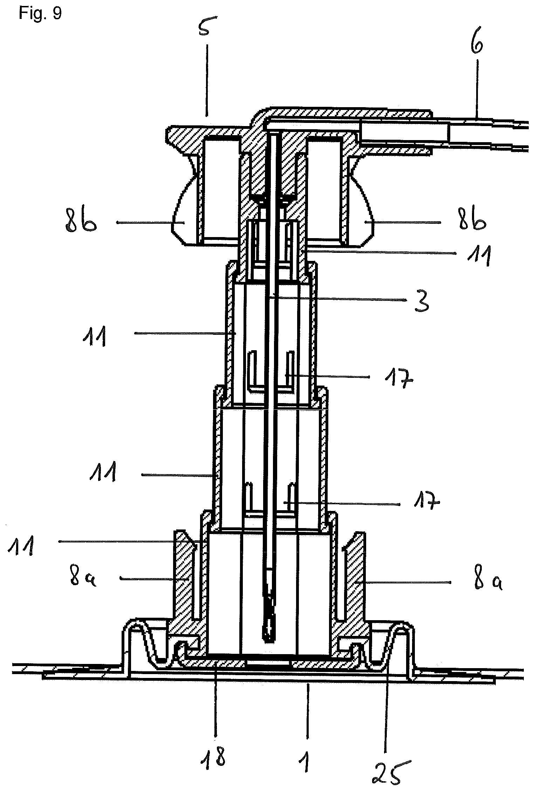

Fig 9 zeigt einen Schnitt durch eine Portkanüle mit ausgefahrenem und eingerasteten Sicherheitsmechanismus. Der Kanülenhalter 5 ist verbunden mit der Kanüle 3 und dem Schlauch 6 und weist im oberen Bereich eine Halterippe auf. Die Gleitrippen 8b befinden sich am Kanülenhalter 5. Das obere Teleskopelement 11 ist durch eine unlösbare Klipsverbindung mit dem Kanülenhalter 5 verbunden. Die Teleskopelemente 11 sind durch Rastelemente 17 miteinander verrastet. Das untere Teleskopelement 11 ist mit der Höhenausgleichsplatte verbunden und weist Klipslaschen 8a, die mit den Gleitrippen 8b zusammenwirken.

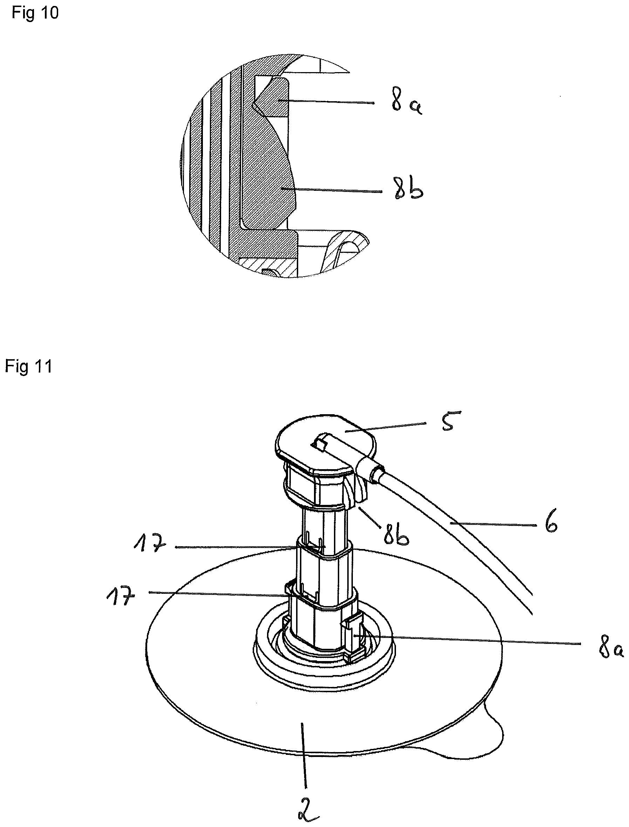

Fig 10 zeigt im Detail das Zusammenwirken von Klipslasche 8a und Gleitrippe 8b.

Fig 11 zeigt eine Ansicht auf die Portkanüle mit ausgefahrenem Sicherheitsmechanismus. Die Rastelemente sind als Klipsnasen und Klipslaschen ausgebildet.

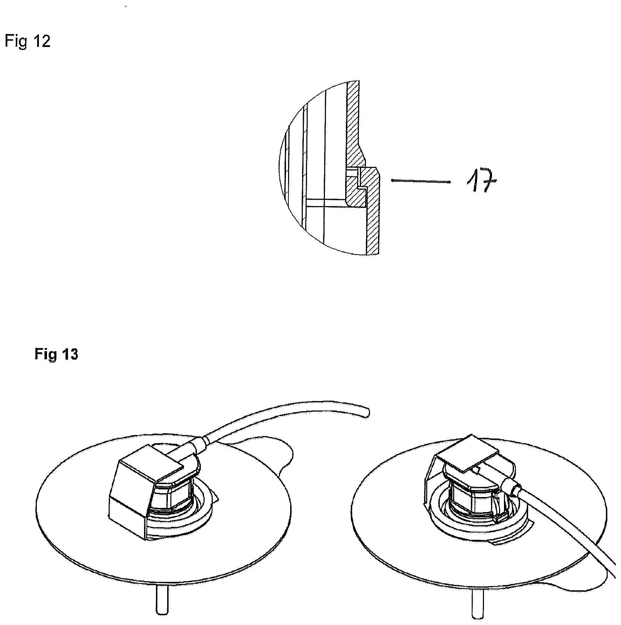

Fig 12 zeigt eine Detailansicht der Verrastung aus Fig 11.

Fig 13 zeigt eine Ausführungsform, in der das Pflaster eine zusätzliche Lasche ausweist, die nach der Anlage über die Portkanüle geklebt werden kann. Diese zusätzliche Fixierung schützt durch einen weiteren Mechanismus vor unbeabsichtigtem Ablösen.

Die in den Beispielen beschriebenen Ausführungsformen wurden einem Vergleichstest mit im Markt befindlichen Sicherheitsportkanülen unterzogen. Ziel dieser Messung war es, die benötigte Kraft zur Auslösung des Sicherheitsmechanismus zu bestimmen.
Messmittel: Erichsen Physimeter 906 MCB - 200N

Die erfindungsgemäßen Beispiele (unterschiedliche Ausführungsformen) erforderten eine Kraft von 9-11N zur Auslösung des Sicherheitsmechanismus.
BBraun Surecan SafeStep: Auszugskraft (Entsichern des Safety Mechanismus) 1,2N.
PFM EZ Huber Safety Infusion Set: Auszugskraft (Entsichern des Safety Mechanismus) 1,75N.

Der Vergleich mit zwei typischen Marktprodukten ergab, dass die benötigte Kraft zum Auslösen des Sicherheitsmechanismus in den erfindungsgemäßen Beispielen um mindestens einen Faktor 5 größer ist.

Ein erfindungsgemäßes Beispiel mit ausgefahrenem und eingerasteten Teleskop hielt einem Druck, entlang der Kanülenachse von bis zu 63N Stand.

Werteermittlung mittels Druckversuch mit "TesT Universalprüfmaschine 106,2 KN. H".

Eine erfindungsgemäße Ausführung mit umlaufender Stopprippe in den Teleskopelementen hielt einem Zug von bis zu 40 N stand, ohne dass die Elemente in der ausgezogenen Form auseinander gezogen werden konnten.

Werteermittlung mittels Zugversuch mit "TesT Universalprüfmaschine 106,2 KN . H".

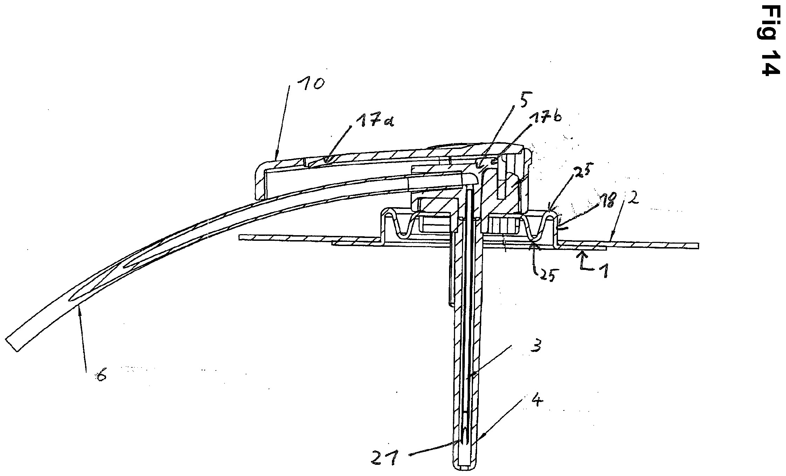

Fig. 14 zeigt eine Schnittdarstellung einer solchen Portkanüle mit dem Hebel 10 in Punktionsstellung. Zu erkennen sind die Auflage 1, die von einer Klebeplatte 2 umgeben ist, die Basis 18, die ein Höhenausgleichselement 25 beinhaltet, die Kanüle 3 mit der Kanülenspitze 21, die von einem Kanülenschutz 4 umgeben ist, der Hebel 10, der Kanülenhalter 5, ein Schlauch 6 und erste Rastelemente 17a, 17b für die Verrastung in zurückgezogener Stellung. Die Kanüle 3 ist mit dem Schlauch 6 verbunden. Diese Verbindung liegt innerhalb des Kanülenhalters 5, an dem die Kanüle 3 und der Schlauch 6 befestigt sind. Der Kanülenhalter 5 ist mit der Basis 18 über ein U-Profil verbunden, das sich über den Hebel 10 weiter erstreckt. Die Auflage 1 ist mit der Basis 18 und der Klebeplatte 2 verbunden. Dabei ist in die Basis 18 durch die wellenförmige Ausbildung das Höhenausgleichselement 25 integriert, das zum Ausgleich der Punktionstiefe dient.

Zur Anwendung wird zunächst der Kanülenschutz 4 entfernt und wird anschließend mit der Kanüle 3 bzw. der Kanülenspitze 21 punktiert. Am Ende dieses Vorganges kommt die Portkanüle mit der Auflage 1 auf dem Patienten bzw. auf den Port zu liegen. Anschließend wird die Portkanüle mit der Klebeplatte 2 am Patienten befestigt. Nun kann durch die zwischen Schlauch 6 und Kanüle 3 bestehende Verbindung eine Substanz in den Port eingebracht werden. Dabei kann das Höhenausgleichselement 25 Relativbewegungen zwischen Kanüle 3 und Auflage 1 sowie Abweichungen der Länge der Kanüle 3 von der Tiefe des Portbodens ausgleichen.

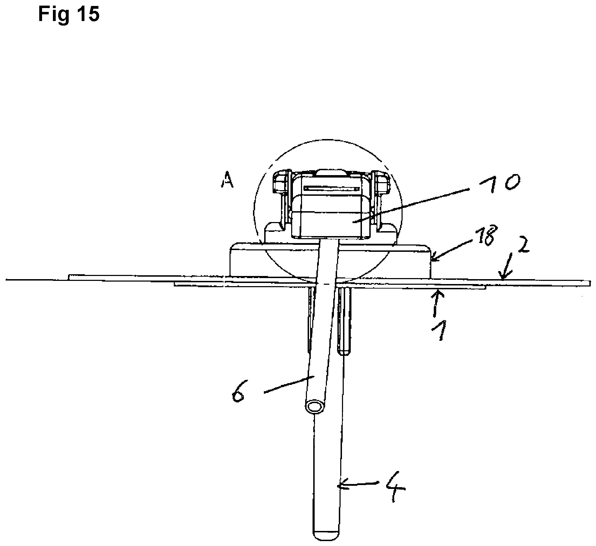

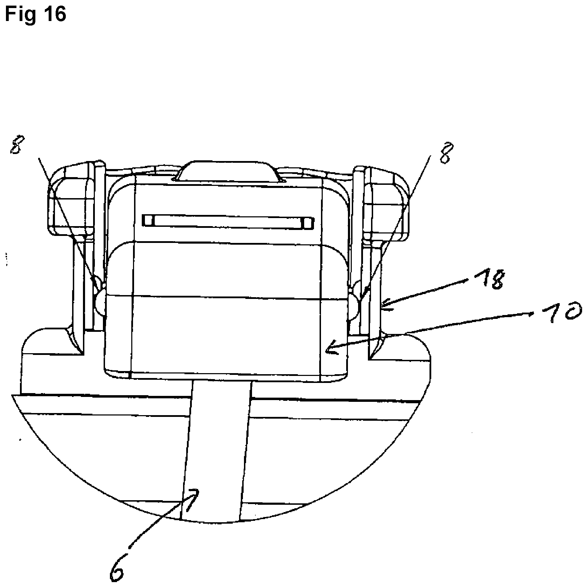

Fig. 15 zeigt noch einmal die Portkanüle in rückwärtiger Ansicht im Auslieferungszustand mit Kanülenschutz 4. Der in Fig. 15 mit einem Kreis und dem Bezugszeichen "A" gekennzeichnete Ausschnitt ist vergrößert in Fig. 16 wiedergegeben. Fig. 16 zeigt somit eine rückwärtige Ansicht eines Ausschnittes der Portkanüle. Zu erkennen sind der Schlauch 6 und der Hebel 10, sowie die Basis 18. An der Basis 18 und dem Hebel 10 sind jeweils zweite Rastelemente 8 für die Verrastung in Punktionsstellung angebracht. Diese zweiten Rastelemente 8 sichern die Punktionsstellung und ermöglichen ein zuverlässiges Punktieren, ohne dass sich die Kanüle 3 gegenüber den anderen Elementen der Portkanüle, insbesondere der Auflage 1 und der Basis 18, wesentlich bewegen kann. Kleinere Bewegungen, die durch das Höhenausgleichselement 25 ermöglicht werden, sind für die Punktion unschädlich.

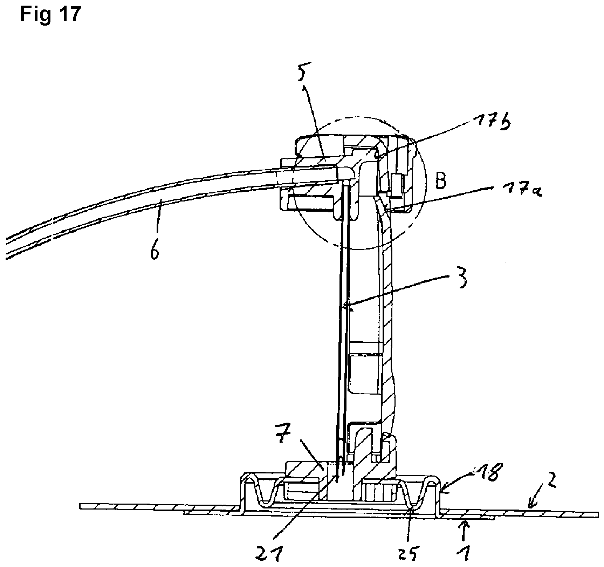

Fig. 17 zeigt den Zustand der Portkanüle in zurückgezogener Stellung. Zum Erreichen dieser Stellung wird ausgehend von der Darstellung in Fig. 14 zunächst der Hebel 10 um 90° nach oben geschwenkt. Sodann wird die Kanüle 3 mit dem Kanülenhalter 5 und dem Schlauch 6 unterstützt durch die Führung durch das U-Profil am Kanülenhalter 5 und dem Hebel 10 den Hebel 10 entlang nach oben gezogen. Dadurch wird die Kanüle 3 aus dem Port bzw. dem Patienten herausgezogen. Dieser Zustand ist in Fig. 17 gezeigt. Zu erkennen ist hier die zurückgezogene Kanüle 3 mit der in den Sicherungsabschnitt 7 verbrachten Kanülenspitze 21. Aus der Darstellung wird deutlich, dass die Kanülenspitze 21 so vom Sicherungsabschnitt 7 umgeben ist, dass ein unabsichtliches Berühren der Kanülenspitze 21 nicht möglich ist. Dabei ist die Kanüle 3 zwar nicht vollständig umschlossen, von der Seite jedoch und insbesondere an der Kanülenspitze 21 ausreichend umgeben, so dass eine Berührung der Kanülenspitze 21 zuverlässig verhindert werden kann. Dabei verhindern die als Rastnase 17b am Kanülenhalter 5 und Rastlasche 17a an dem Hebel 10 ausgebildeten ersten Rastelemente für die Verrastung in zurückgezogener Stellung ein Bewegen der Kanülenspitze 21 aus dem Sicherungsabschnitt 7 heraus. Die ersten Rastelemente 17a, 17b verhindern dabei nicht jede Bewegung der Kanüle 3 beziehungsweise des Kanülenhalters 5 oder der Kanülenspitze 21. Sie lassen vielmehr ein gewisses Spiel zu, damit eine Bewegung in Richtung der zurückgezogenen Stellung auch über den Punkt der Verrastung hinaus ermöglicht wird. Dies erhöht die Anwendungssicherheit, da dadurch die Wahrscheinlichkeit einer zuverlässigen Verrastung durch den Anwender erhöht wird.

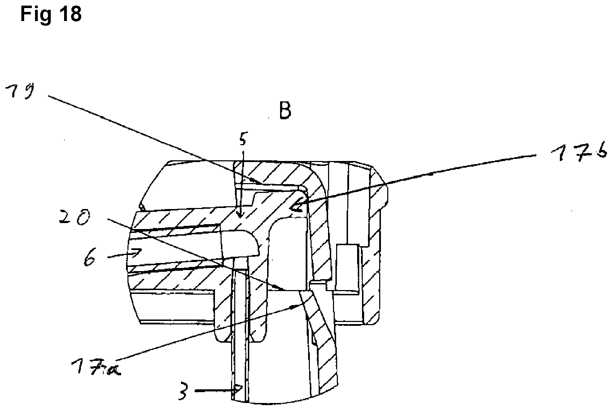

Der in Fig. 17 durch einen Kreis und mit dem Bezugszeichen "B" gekennzeichnete Bereich ist in Fig. 18 vergrößert dargestellt.

Fig. 18 zeigt somit einen Ausschnitt aus Fig. 17 und zeigt den Endbereich des Hebels 10 mit dem Kanülenhalter 5. Zu erkennen ist hier noch einmal deutlich, dass der Kanülenhalter 5 als erstes Rastelement für die Verrastung in zurückgezogener Stellung eine Rastnase 17b aufweist, die mit der am Hebel 10 als weiteres erstes Rastelement vorgesehenen Rastlasche 17a für die Verrastung in zurückgezogener Stellung derart zusammenwirkt, dass ein Bewegen der Kanüle 3 in Richtung Punktionsstellung mit einem gewissen Spiel verhindert wird. Dieses Spiel zwischen der oberen Begrenzung 19 und der unteren Begrenzung 20 ist in Fig. 18 gut zu erkennen, da der Kanülenhalter 5 bis zum oberen Anschlag an der oberen Begrenzung 19 heraufgezogen wurde. Von dort an hat er nun ein gewisses Spiel nach unten, bis die Rastnase 17b als eines der ersten Rastelemente für die Verrastung in zurückgezogener Stellung am Kanülenhalter 5 mit der Rastlasche 17a für die Verrastung in zurückgezogener Stellung am Hebel 10 an der unteren Begrenzung 20 in Eingriff gebracht wird. Ein solches Spiel ist jedoch nicht weiter schädlich und für eine sichere Verrastung in der zurückgezogenen Stellung sogar förderlich, da der Anwender in einem solchen Fall die Rastnase 17b problemlos und sicher über die Rastlasche17a für die Verrastung in zurückgezogener Stellung am Hebel 10 hinweg bewegen kann.

Der Sicherungsabschnitt 7 ist dementsprechend groß ausgebildet, so dass auch beim Ausnutzen des vorhandenen Spiels eine Gefahr der Berührung der Kanülenspitze 21 nicht gegeben ist.

## Patentansprüche

1. Portkanüle aufweisend
- eine Auflage (1) zur Auflage der Portkanüle am Patienten,
- eine Zuführung (6) und
- eine Kanüle (3) mit einer Spitze (21), wobei die Kanüle (3) in mindestens zwei Positionen, eine Punktionsstellung und eine zurückgezogene Stellung, verbringbar ist, wobei die Kanüle (3) an einem Kanülenhalter (5) befestigt ist, an dem vorzugsweise auch die Zuführung (6) befestigt ist, der mit einer Vorrichtung zur Arretierung des Kanülenhalters in der Punktionsstellung, die mit der Auflage (1) verbunden ist, lösbar verbunden ist,
- einen Sicherungsabschnitt (7), der die Spitze (21) der Kanüle (3) mindestens in der zurückgezogenen Stellung abschirmt und so eine Nadelstichschutzvorrichtung bildet,
**dadurch gekennzeichnet, dass** zweite Rastelemente (8), die die Kanüle (3) durch den Kanülenhalter (5) in der Punktionsstellung verrasten, vorgesehen sind und die Kraft, die zur Lösung der Verbindung zwischen Kanülenhalter (5) und der Vorrichtung zur Arretierung des Kanülenhalters nötig ist, mindestens 5 N beträgt.

2. Portkanüle nach Anspruch 1, **dadurch gekennzeichnet, dass** die Portkanüle ein zwischen Auflage (1) und Kanüle (3) positioniertes, flexibles und/oder dehnbares Höhenausgleichselement (25) aufweist.

3. Portkanüle nach einem der Ansprüche 1 oder 2, **dadurch gekennzeichnet, dass** erste Rastelemente (17), die die Kanüle (3) in der zurückgezogenen Stellung verrasten, vorgesehen sind.

4. Portkanüle nach Anspruch 4, **dadurch gekennzeichnet, dass** die zweiten Rastelemente (8) bei der Punktion gesichert sind.

5. Portkanüle nach einem der vorstehenden Ansprüche, **dadurch gekennzeichnet, dass** sich die Kanüle (3) zur Verbringung in die mindestens zwei Positionen relativ zu einer Basis (18) bewegen lasst, die mit der Auflage (1) fest verbunden ist.

6. Portkanüle nach einem der vorstehenden Ansprüche, **dadurch gekennzeichnet, dass** sie eine Klebeplatte (2) zur Befestigung der Auflage (1) am Patienten aufweist.

7. Portkanüle nach einem der Ansprüche 1 bis 7, **dadurch gekennzeichnet, dass** sie mindestens zwei Teleskopsegemente (11) aufweist, die gegeneinander beweglich und untereinander geführt ausgebildet sind, wobei die Kanüle (3) mit einem der Teleskopsegmente (11) verbunden ist und ein anderes Teleskopsegment mit der Auflage (1) verbunden ist und wobei die Teleskopsegmente (11) in der Punktionsstellung weitestgehend ineinander geschoben und in der zurückgezogenen Stellung weiter auseinander gezogen sind.

8. Portkanüle nach Anspruch 8, **dadurch gekennzeichnet, dass** eines der Teleskopsegmente (11) mit der Basis (18) verbunden ist oder die Basis (18) bildet und/oder eines der Teleskopsegmente (11) mit dem Kanülenhalter (5) verbunden ist oder diesen bildet.

9. Portkanüle nach einem der Ansprüche 8 oder 9, **dadurch gekennzeichnet, dass** jedes Teleskopsegment (11) mindestens ein erstes Rastelement (17) für die Verrastung in der zurückgezogenen Stellung aufweist.

10. Portkanüle nach Anspruch 10, **dadurch gekennzeichnet, dass** die Teleskopelemente (11) ineinander geschachtelt sind und über das erste Rastelement mit jedem benachbarten Teleskopsegment verrastbar ist.

11. Portkanüle nach Anspruch 11, **dadurch gekennzeichnet, dass** die ineinander geschachtelten Teleskopsegmente (11) jeweils ein so geringes Spiel und eine präzise Auslegung und Formgebung der aneinander vorbei bewegenden Wände aufweisen, dass ein Verdrehen des Kanülenhalters (5) und/oder Verkanten der Nadel (3) beim Bewegen in die zurückgezogenen Stellung und/oder in der zurückgezogenen Stellung verhindert wird.

12. Portkanüle nach einem der Ansprüche 8 bis 12, **dadurch gekennzeichnet, dass** die Kanüle (3) innerhalb der Teleskopsegmente (11) angeordnet ist.

13. Portkanüle nach einem der Ansprüche 8 bis 13, **dadurch gekennzeichnet, dass** vier Teleskopsegmente (11), insbesondere aus Hohlquadern mit jeweils zwei ausgesparten Seitenflächen gebildete, vorgesehen sind.

14. Portkanüle nach einem der Ansprüche 8 bis 14, **dadurch gekennzeichnet, dass** das am nächsten zur Auflage (1) hin liegende Teleskopsegment (11) den Sicherungsabschnitt (7) bildet.

## Claims

1. Port needle having
- a bearing (1) for resting the port needle on the patient,
- a feed line (6), and
- a needle (3) with a tip (21), which needle (3) can be brought into at least two positions, namely a puncture position and a retracted position, wherein the needle (3) is secured on a needle holder (5), on which the feed line (6) is preferably also secured, which needle holder is releasably connected to a device for locking the needle holder in the puncture position, which device is connected to the bearing (1),
- a safety section (7), which shields the tip (21) of the needle (3) at least in the retracted position and thus forms a needle-prick protection device,
**characterized in that** second latching elements (8) are provided, which latch the needle (3) in the puncture position, and the force needed to release the connection between the needle holder (5) and the device for locking the needle holder is at least 5 N.

2. Port needle according to Claim 1, **characterized in that** the port needle has a flexible and/or extensible height compensation element (25) positioned between bearing (1) and needle (3).

3. Port needle according to either of Claims 1 and 2, **characterized in that** first latching elements (17) are provided, which latch the needle (3) in the retracted position.

4. Port needle according to Claim 3, **characterized in that** the second latching elements (8) are secured during the puncture.

5. Port needle according to one of the preceding claims, **characterized in that** the needle (3), in order to be brought into the at least two positions, can be moved relative to a base (18), which is rigidly connected to the bearing (1).

6. Port needle according to one of the preceding claims, **characterized in that** it has an adhesion plate (2) for securing the bearing (1) on the patient.

7. Port needle according to one of Claims 1 to 6, **characterized in that** it has at least two telescope segments (11), which are designed to be movable relative to each other and guided by each other, wherein the needle (3) is connected to one of the telescope segments (11), and another telescope segment is connected to the bearing (1), and wherein the telescope segments (11) are pushed as far as possible one inside the other in the puncture position and are pulled farther apart in the retracted position.

8. Port needle according to Claim 7, **characterized in that** one of the telescope segments (11) is connected to the base (18) or forms the base (18) and/or one of the telescope segments (11) is connected to the needle holder (5) or forms the latter.

9. Port needle according to either of Claims 7 and 8, **characterized in that** each telescope segment (11) has at least one first latching element (17) for the latching in the retracted position.

10. Port needle according to Claim 9, **characterized in that** the telescope elements (11) are stacked one inside the other and can be latched onto each adjacent telescope segment via the first latching element.

11. Port needle according to Claim 10, **characterized in that** the telescope segments (11) stacked one inside the other each have such little play, and a precise configuration and shaping of the walls moving past each other, that a twisting of the needle holder (5) and/or jamming of the needle (3) is prevented during the movement into the retracted position and/or in the retracted position.

12. Port needle according to one of Claims 7 to 11, **characterized in that** the needle (3) is arranged inside the telescope segments (11).

13. Port needle according to one of Claims 7 to 12, **characterized in that** four telescope segments (11) are provided, which are formed in particular from hollow cuboids with in each case two cut-out side faces.

14. Port needle according to one of Claims 7 to 13, **characterized in that** the telescope segment (11) lying nearest to the bearing (1) forms the safety section (7).

## Revendications

1. Canule pour chambre implantable présentant
- un appui (1) pour placer la canule pour chambre implantable sur le patient,
- une arrivée (6) et
- une canule (3) avec une pointe (21), dans laquelle la canule (3) peut être placée dans au moins deux positions, une position de ponction et une position rétractée, dans laquelle la canule (3) est fixée à un support de canule (5), sur lequel l'arrivée (6) est de préférence également fixée, qui est assemblé de façon séparable à un dispositif pour bloquer le support de canule dans la position de ponction qui est assemblé à l'appui (1),
- une partie de sécurité (7), qui protège la pointe (21) de la canule (3) au moins dans la position rétractée et forme ainsi un dispositif de protection contre une piqûre par l'aiguille,
**caractérisée en ce qu'**il est prévu des deuxièmes éléments d'encliquetage (8), qui calent la canule (3) dans la position de ponction au moyen du support de canule (5), et la force qui est nécessaire pour lever l'assemblage entre le support de canule (5) et le dispositif pour bloquer le support de canule vaut au moins 5 N.

2. Canule pour chambre implantable selon la revendication 1, **caractérisée en ce que** la canule pour chambre implantable présente un élément de compensation de hauteur flexible et/ou extensible (25) positionné entre l'appui (1) et la canule (3).

3. Canule pour chambre implantable selon une des revendications 1 ou 2, **caractérisée en ce qu'**il est prévu des premiers éléments d'encliquetage (17), qui calent la canule (3) dans la position rétractée.

4. Canule pour chambre implantable selon la revendication 3, **caractérisée en ce que** les deuxièmes éléments d'encliquetage (8) sont bloqués lors de la ponction.

5. Canule pour chambre implantable selon l'une quelconque des revendications précédentes, **caractérisée en ce que** pour le placement dans lesdites au moins deux positions la canule (3) peut être déplacée par rapport à une base (18), qui est fixée à l'appui (1).

6. Canule pour chambre implantable selon l'une quelconque des revendications précédentes, **caractérisée en ce qu'**elle présente une plaque adhésive (2) pour la fixation de l'appui (1) sur le patient.

7. Canule pour chambre implantable selon l'une quelconque des revendications 1 à 6, **caractérisée en ce qu'**elle présente au moins deux segments télescopiques (11), qui sont mobiles l'un par rapport à l'autre et qui sont guidés l'un en dessous de l'autre, dans laquelle la canule (3) est reliée à un des segments télescopiques (11) et un autre segment télescopique est relié à l'appui (1), et dans laquelle les segments télescopiques (11) sont très largement glissés l'un dans l'autre dans la position de ponction et sont plus largement tirés l'un hors de l'autre dans la position rétractée.

8. Canule pour chambre implantable selon la revendication 7, **caractérisée en ce qu'**un des segments télescopiques (11) est relié à la base (18) ou forme la base (18) et/ou un des segments télescopiques (11) est relié au support de canule (5) ou forme celui-ci.

9. Canule pour chambre implantable selon une des revendications 7 ou 8, **caractérisée en ce que** chaque segment télescopique (11) présente au moins un premier élément d'encliquetage (17) pour le blocage dans la position rétractée.

10. Canule pour chambre implantable selon la revendication 9, **caractérisée en ce que** les éléments télescopiques (11) sont emboîtés l'un dans l'autre et peuvent être encliquetés avec chaque segment télescopique voisin au moyen du premier élément d'encliquetage.

11. Canule pour chambre implantable selon la revendication 10, **caractérisée en ce que** les segments télescopiques (11) emboîtés l'un dans l'autre présentent respectivement un jeu tellement faible et une conception et une configuration tellement précises des parois se déplaçant l'une le long de l'autre, qu'une rotation du support de canule (5) et/ou un coincement de l'aiguille (3) lors du déplacement vers la position rétractée et/ou dans la position rétractée soit empêché(e).

12. Canule pour chambre implantable selon l'une quelconque des revendications 7 à 11, **caractérisée en ce que** la canule (3) est disposée à l'intérieur des segments télescopiques (11).

13. Canule pour chambre implantable selon l'une quelconque des revendications 7 à 12, **caractérisée en ce qu'**il est prévu quatre segments télescopiques (11), en particulier formés à partir de parallélépipèdes creux avec chaque fois deux faces latérales évidées.

14. Canule pour chambre implantable selon l'une quelconque des revendications 7 à 13, **caractérisée en ce que** le segment télescopique (11) disposé le plus près de l'appui (1) forme la partie de sécurité (7).
